Europäisches Patentamt

European Patent Office

Office européen des brevets

(11) Veröffentlichungsnummer: **0 065 594**
A2

(12) ## EUROPÄISCHE PATENTANMELDUNG

(21) Anmeldenummer: 81105163.0

(22) Anmeldetag: 03.07.81

(51) Int. Cl.³: **H 05 G 1/02**
H 05 G 1/46, A 61 B 6/00

(30) Priorität: 27.05.81 DE 3121227

(43) Veröffentlichungstag der Anmeldung:
01.12.82 Patentblatt 82/48

(84) Benannte Vertragsstaaten:
AT BE CH FR GB IT LI LU NL SE

(71) Anmelder: Mediprix AG
Hohenrainstrasse 9
CH-6280 Hochdorf(CH)

(72) Erfinder: Laupper, Rudolf
Schlüsselrain 3
CH-6024 Hildisrieden(CH)

(74) Vertreter: Riebling, Günter, Dr. Ing. et al,
Rennerle 10 Postfach 3160
D-8990 Lindau(DE)

(54) Stativ für ein Röntgengerät.

(57) Die Erfindung betrifft ein Stativ für ein Röntgengerät mit einer vertikalen Säule, an der in vertikaler Richtung verschiebbar ein Vertikalwagen angebracht ist, an dem ein Querbalken drehbar gelagert ist, an dem schwenkbar und in dessen Längsrichtung verschiebbar eine Buckywand und davon getrennt ebenfalls schwenkbar und in dessen Längsrichtung verschiebbar eine Röntgenröhre vorgesehen sind, wobei gegebenenfalls eine wahlweise einschaltbare Steuerung für Antriebe dieser Bauelemente vorgesehen ist, nach Patentanmeldung P 30 10 592.1, dadurch gekennzeichnet, daß die Steuerung die Buckywand 22 in einer einmal eingestellten Höhe hält und gegebenenfalls die Röntgenröhre 26 in eine vorgegebene Position fährt.

EP 0 065 594 A2

Croydon Printing Company Ltd.

- 1 -

Stativ für ein Röntgengerät
---------------------------

Zusatz zu Patentanmeldung P 30 10 592.1

Die Erfindung betrifft ein Stativ für ein Röntgengerät mit einer vertikalen Säule, an der in vertikaler Richtung verschiebbar ein Vertikalwagen angebracht ist, an dem ein Querbalken drehbar gelagert ist, an dem schwenkbar und in dessen Längsrichtung verschiebbar eine Buckywand und davon getrennt ebenfalls schwenkbar und in dessen Längsrichtung verschiebbar eine Röntgenröhre vorgesehen sind, wobei gegebenenfalls eine wahlweise einschaltbare Steuerung für Antriebe dieser Bauelemente vorgesehen ist, nach Patentanmeldung P 30 10 592.1.

Ein solches Stativ ist Gegenstand der älteren Patentanmeldung P 30 10 592.1. Dieses hat sich bewährt, insbesondere, weil die verschiedenen Positionen der Buckywand relativ zur Röntgenröhre je nach den jeweiligen Verhältnissen von Hand oder über eine Steuerung optimal eingestellt werden können. Beim Gegenstand dieser älteren Patentanmeldung wird hierdurch eine optimale Strahlungsintensität und Strahlungsbelastung des Patienten erzielt, welche Größen bekanntlich vom jeweils zu untersuchenden Patienten, dem zu untersuchenden Gebiet und anderen Parametern abhängen.

Der vorliegenden Erfindung liegt die Aufgabe zugrunde, dieses bereits vorgeschlagene Stativ hinsichtlich seines Bedienungskomforts und seiner Bedienungssicherheit weiterhin zu verbessern.

Ausgehend von einem Stativ der eingangs genannten Art gelingt dies gemäß der Erfindung dadurch, daß die Steuerung die Buckywand in einer einmal eingestellten Höhe hält und gegebenenfalls die Röntgenröhre in eine vorgegebene Position fährt.

Die Buckywand verbleibt somit auf einer einmal eingestellten Höhe dicht unter der Arbeitsfläche einer Liege für den zu untersuchenden Patienten. Die jeweilige Distanz zwischen der Buckywand und der Röntgenröhre wird durch eine entsprechende Einstellung der Röntgenröhre herbeigeführt. Diese Einstellungen erfolgen vorzugsweise automatisch. Die Vertikalbewegung des Vertikalwagens ist hierbei für Handbetrieb gesperrt.

In diesem Zusammenhang wird es bevorzugt, wenn das Bett an einer seiner Längsseiten offen ist. Das Bett mit dem darauf befindlichen Patienten kann somit leicht seitlich über die in der eingestellten Höhe verbleibende Buckywand gefahren werden.

Bezüglich der Schwenklage des Querbalkens wird es bevorzugt, wenn dieser entweder manuell in eine gewünschte Schwenklage gebracht werden kann oder über die selbsttätige Steuerung entweder in die vertikale oder waagerechte Position, von der sie dann von Hand in eine gewünschte Stellung verschwenkt werden kann. Aus dieser Stellung kann sie dann über die Automatik wieder in die vertikale oder lotrechte Ausgangslage verfahren werden.

Die Bedienung wird weiterhin vereinfacht, wenn die Steuerung den Vertikalwagen in eine Mittelstellung an der Säule fährt und die Aufnahmedistanz auf einen vorgegebenen Abstand bringt, der beispielsweise einen Meter beträgt. Aus diesen Mittellagen heraus lassen sich sehr schnell die jeweils gewünschten Positionen, vorzugsweise von Hand, anfahren, weil von dort aus nur noch geringe Wege zurückgelegt werden müssen.

Aus demselben Grunde wird es bevorzugt, wenn die Buckywand über die Steuerung geneigt werden kann,

Es erhöht ebenfalls den Bedienungskomfort, wenn im Querwagen der Buckywand ein Lautsprecher angeordnet ist, der zur besseren Verständigung mit der gerade untersuchten Person dient.

Es dient weiterhin einer Bedienungsvereinfachung, wenn die Röntgenröhre an einem Querwagen befestigt ist, dessen Position über die Steuerung zur Buckywand seitlich einstellbar ist.

Auch die Höhenlage des Querbalkens ist über die Steuerung vorzugsweise einstellbar.

Bezüglich des Drehantriebes für den Querbalken wird es bevorzugt, wenn diese über eine in einem Ölbad laufende Sicherheits-Rutschkupplung erfolgt. Eine solche Kupplung ist sehr wartungsarm und kann von außen in ihrer Kupplungswirkung eingestellt werden, so daß die jeweils gewünschte Position sehr sanft angefahren wird.

Es ist ebenfalls eine sehr wartungsarme und trotzdem genaue und sichere Anordnung getroffen, wenn die Antriebe für die Verschiebungen über einen oder mehrere Getriebemotoren mit nachgeschalteter Kette erfolgen.

Die Erfindung wird im folgenden an Hand von Ausführungsbeispielen näher erläutert, aus denen sich weitere wichtige Merkmale ergeben.

Es zeigt:

Fig. 1 eine Seitenansicht der wesentlichen Bauteile des neuartigen Stativs mit einer Liege;

Fig. 2 eine Vorderansicht des Stativs mit Querbalken in verschiedenen Drehlagen;

Fig. 3 eine Ansicht ähnlich Fig. 2 mit verschiedenen Schwenklagen der Buckywand und mit verschiedenen seitlichen Verschiebungslagen der Röntgenröhre;

Fig. 4 eine Vorderansicht des Stativs, wobei verschiedene Höheneinstellungen des Querbalkens angedeutet sind;

Fig. 5 eine Ansicht ähnlich Fig. 4, wobei verschiedene Positionen der Buckywand und der Röntgenröhre längs des Querbalkens dargestellt sind;

Fig. 6 schematisch den Antrieb für die Verschiebung der Röntgenröhre;

Fig. 7 einen Achsialschnitt durch die beim Antrieb nach Fig. 6 verwendete Rutschkupplung;

Fig. 8 - 10 die Führung des Querwagens und den Antrieb der Buckywand in verschiedenen Ansichten, nämlich in Fig. 8 in einer Seitenansicht, in Fig. 9 in einer Stirnansicht von Fig. 8 und in Fig. 10 in einem Schnitt längs der Linie A-A von Fig. 8;

Fig. 11 und 12 die Führung des Vertikalwagens bzw. den Antrieb des Querbalkens.

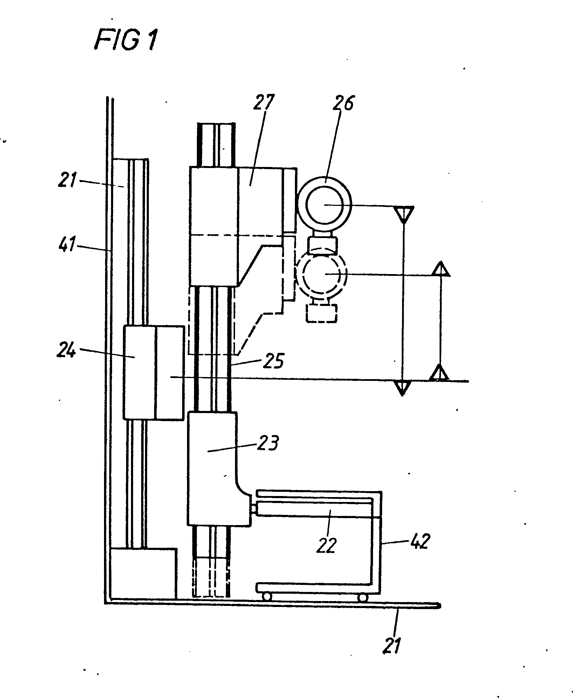

Das in den Figuren gezeigte neuartige Stativ steht auf einer Aufstellfläche 28, und zwar vorzugsweise in der Nähe einer Wand 41 (vergleiche insbesondere Fig. 1). Das Stativ besteht aus einer Wandsäule 21, die lotrecht an der Wand 41 montiert ist. An der Wandsäule 21 ist ein Vertikalwagen 24 in lotrechter Richtung verschiebbar, und zwar vorzugsweise über einen gesteuerten Motor.

Der Vertikalwagen 24 trägt einen an ihm drehbar gelagerten Querbalken 25. Auf dem Querbalken sind in dessen Längsrichtung verschiebbar ein linker Querwagen 23 und ein rechter Querwagen 27. Am linken Querwagen 23 ist schwenkbar gelagert eine Buckywand 22, auch Auftreffschirm für die Röntgenstrahlen genannt.

Am rechten Querwagen 27 ist eine Röntgenröhre 26 befestigt, die in Richtung senkrecht zur Papierebene der Fig. 1 an diesem Querwagen verschoben werden kann.

Fig. 1 zeigt außerdem eine Liege 42 für eine mit dem Röntgenapparat zu untersuchende Person. Diese ist an einer Seite frei, so daß sie über die Buckywand 22 gefahren werden kann, wie in Fig. 1 gezeigt.

Fig. 1 erläutert, daß die Buckywand bei allen Distanzen zwischen ihr und der Röntgenröhre 26 auf der gleichen Höhe bleibt. Wird über die in der Hauptpatentanmeldung näher beschriebene Automatik die Tischhöhe der Liege 42 eingestellt, so pendelt sich die Buckywand immer auf die gleiche Höhe ein und das Liebebett kann ohne Schwierigkeiten über die Buckywand verschoben werden.

Die Vertikalbewegung des Vertikalwagens 24 ist jetzt
für Handbetrieb gesperrt.

Wird durch einen Befehlsgenerator oder durch einen
manuellen Befehl eine andere Aufnahmedistanz vorgegeben,
so setzen sich der Vertikalwagen und die Röntgenröhre
gleichzeitig in Bewegung, bis die gewünschte Aufnahmedistanz erreicht ist. Auch hierbei bleibt die Buckywand
stehen.

Wird die Tischhöhe über die Steuerung schon in waagerechter Lage des Querbalkens abgesenkt, so bleibt dieser
Befehl gespeichert, jedoch ohne Auswirkung auf die Höhe
der Buckywand. Wird dann der Querbalken in die senkrechte
Lage gebracht, so tritt die Automatik für die gespeicherte Tischhöhe in Aktion und bringt die Buckywand selbsttätig in die richtige Höhe.

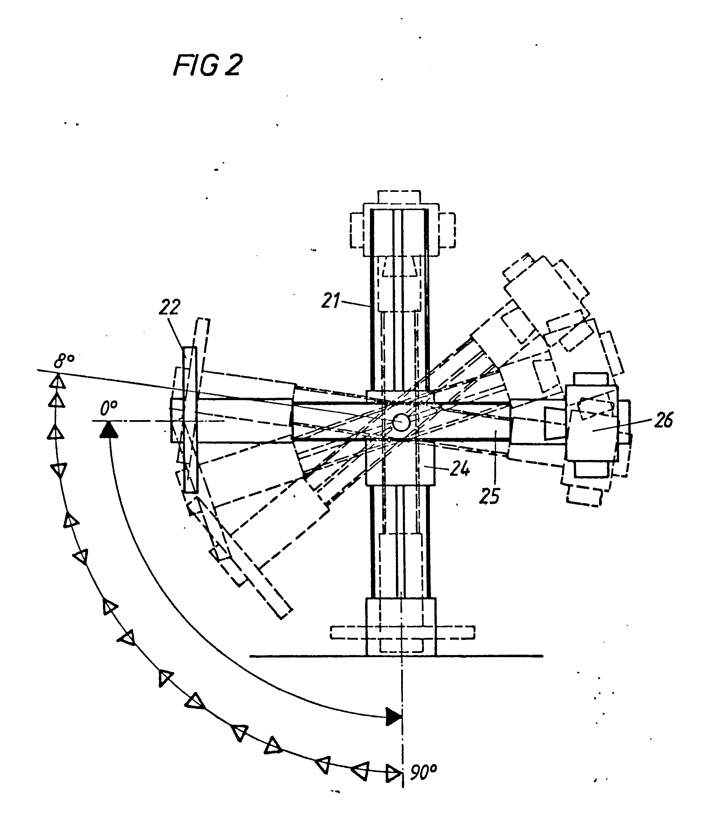

Fig. 2 zeigt, daß der Querbalken 25 elektrisch gedreht
werden kann, wozu zwei Möglichkeiten zur Verfügung
stehen: Er kann entweder von Hand in jede gewünschte
Schräglage von minus 8 Grad bis plus 90 Grad gedreht
werden, oder er wird selbsttätig aus der waagerechten
Lage (0 Grad) oder der senkrechten Lage (90 Grad) gedreht. Beim automatischen Drehen fährt der Vertikalwagen 24 ist eine Mittelstellung und auf die übliche
Aufnahmedistanz von etwa einem Meter. Aus jeder beliebigen Schräglage kann der Querbalken automatisch in die
waagerechte oder senkrechte Lage gefahren werden.

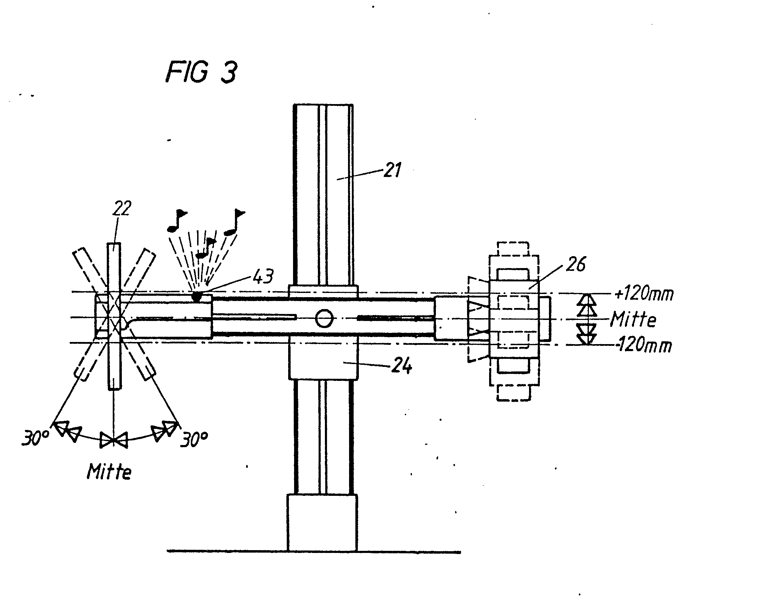

Fig. 3 zeigt, daß die Buckywand nach beiden Seiten
elektrisch um etwa bis zu 30 Grad geneigt werden kann.
Durch eine gesonderte Taste der Steuerung wird die
Buckywand automatisch bei Betätigung der Taste wieder in die Ausgangslage zurückgedreht.

Im Querwagen 23 der Buckywand, also immer in Kopfhöhe des Patienten, ist zur besseren Verständigung ein Lautsprecher 43 vorgesehen, der ebenfalls vom Befehlsgenerator her bedient werden kann.

Die Röntgenröhre 26 kann zum Zentrum der Buckywand seitlich verschoben werden, und zwar beispielsweise beidseits um 120 mm. Durch eine Taste der Steuerung wird die Röhre dann automatisch wieder in ihre Mittellage gebracht.

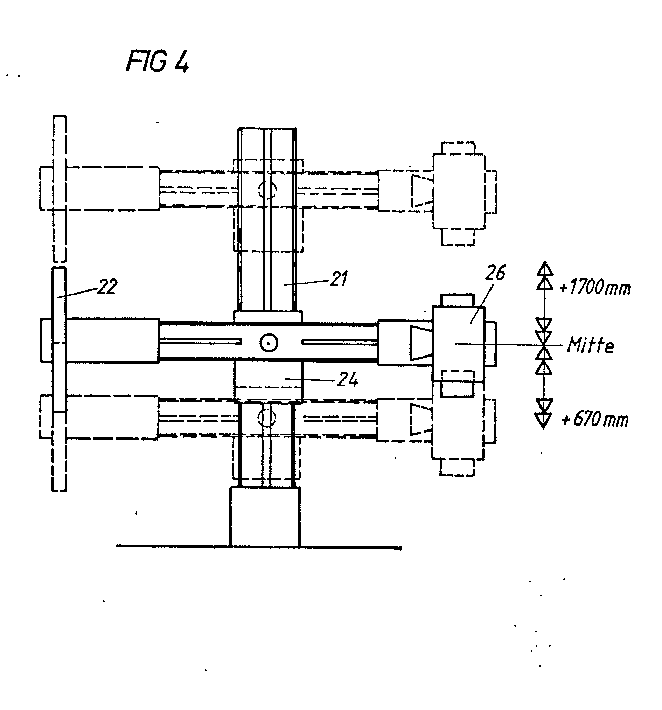

Fig. 4 zeigt, daß der Querbalken durch Tastendruck in der Höhe individuell eingestellt und der Größe des betreffenden Patienten angepaßt werden kann. Beim Drehen des Querbalkens in die senkrechte Lage fährt der Vertikalwagen automatisch in eine Mittelstellung, um Beschädigungen der Röhre oder der Buckywand zu vermeiden.

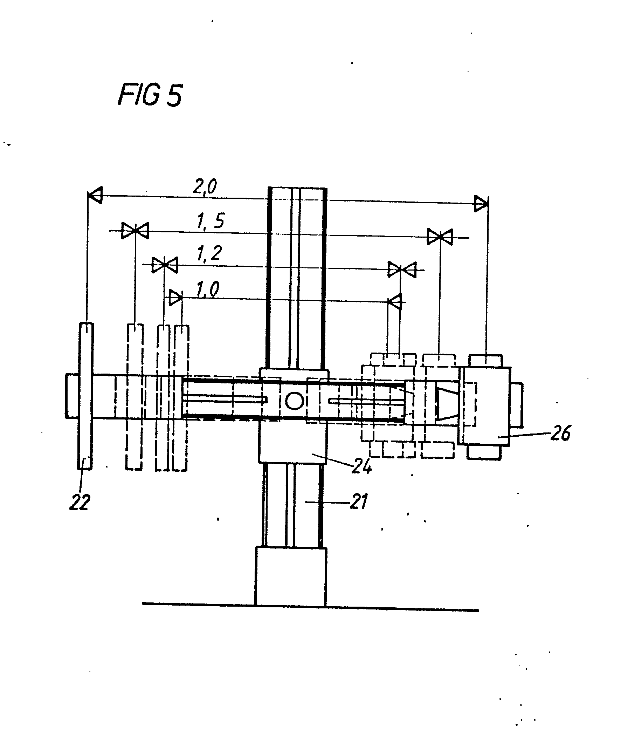

Fig. 5 erläutert, daß die Aufnahmedistanz von Hand oder selbsttätig über die Steuerung auf Fixdistanzen von beispielsweise 1 m, 1,20 m, 1,50 m und 2 m eingestellt werden kann. Die durch die Exposition über den Generator gegebenen Befehle haben hierbei Vorrang gegenüber manuellen Befehlen.

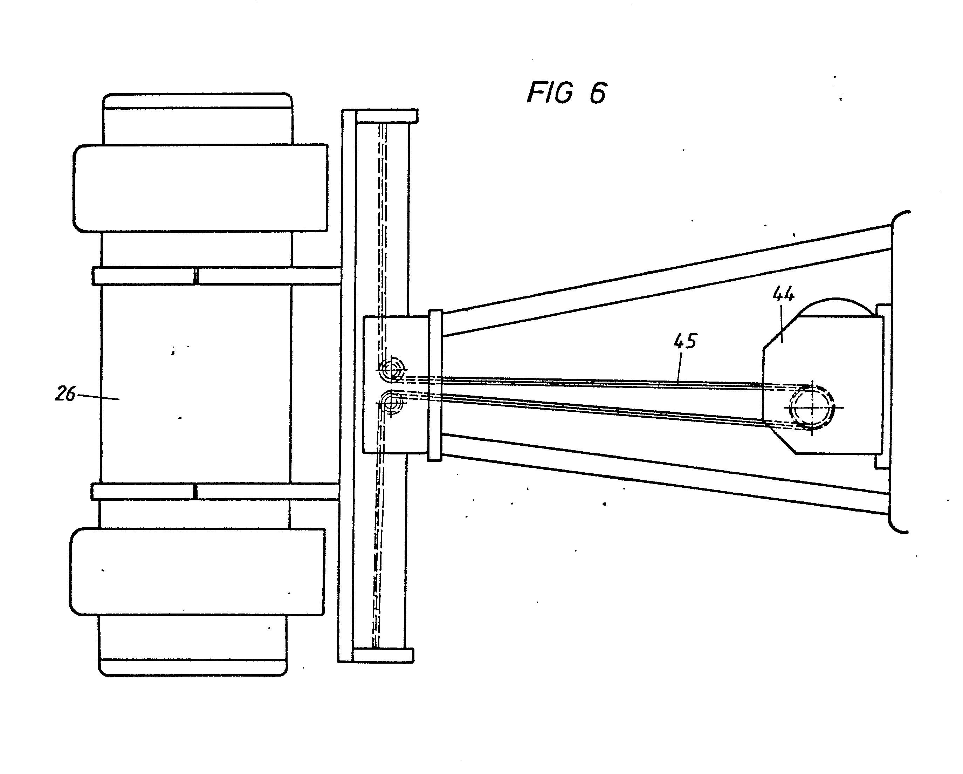

Fig. 6 erläutert den Antrieb für die Verschiebung der Röntgenröhre 26. Hierzu ist ein Getriebemotor 44 vorgesehen, der über eine endlose Antriebskette 45 die Verschiebung der Röntgenröhre, wie in Fig. 2 und/oder Fig. 3 gezeigt, bewirkt.

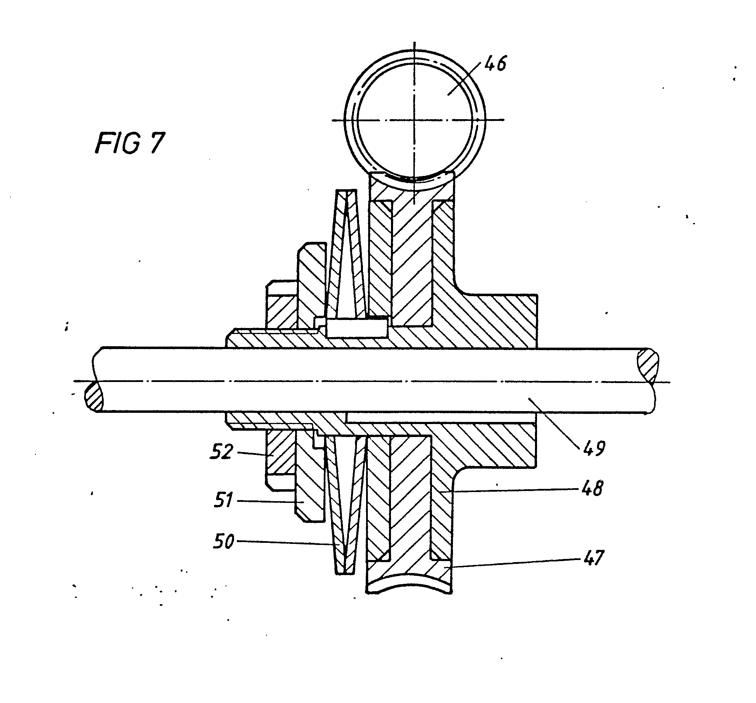

Hierzu erläutert Fig. 7 die im Getriebemotor zum Einsatz kommende Rutschkupplung. Eine Antriebswelle 46 kämmt mit einem Abtriebsrad 47 und dieses wiederum über eine Abtriebsnabe 48 mit einer Abtriebswelle 49. Außerdem sind zwei Kupplungsscheiben 50 vorgesehen, von denen die eine an einer Druckplatte 51 anliegt, an deren freier Fläche wiederum eine Einstellmutter 52 anliegt, die auf die Abtriebsnabe 48 aufgeschraubt ist.

Diese Rutschkupplung ist in das Getriebe des Getriebemotors 44 eingebaut und läuft im Ölbad.

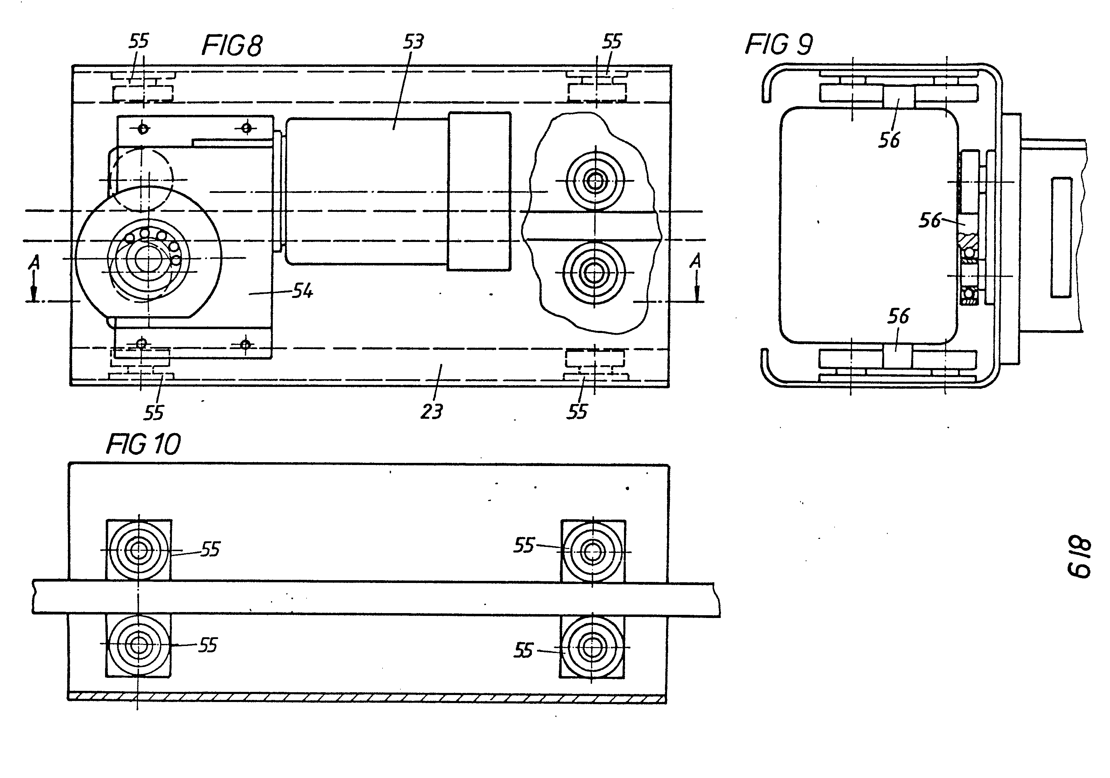

Die Figuren 8 bis 11 zeigen die Führung des Querwagens 23 und den Antrieb der Buckywand 22. Hierfür ist ein Antriebsmotor 53 für die Buckywand mit einem Getriebe 54 vorgesehen. Der Querwagen läuft auf vier Rädern 55.

Dies zeigt auch Fig. 9, die zusätzlich eine rostfreie Stahlschiene 56 erkennen läßt.

Der Querwagen und die Röntgenröhre laufen zwölffach kugelgelagert auf rostfreien Stahlschienen. Die Aufnahmewelle der Buckywand ist vierfach kugelgelagert.

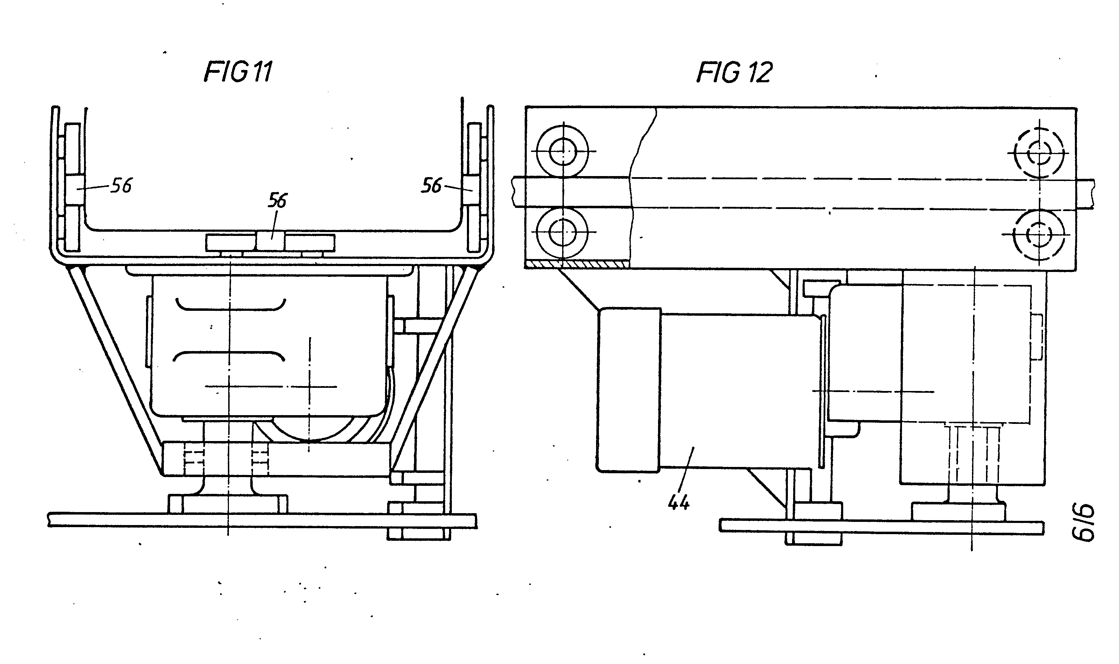

Fig. 11 zeigt, daß der Vertikalwagen zwölffach kugelgelagert auf rostfreien Stahlschienen 56 läuft. Die Aufnahmewelle für den Querbalken ist vierfach kugelgelagert.

Fig. 12 zeigt nochmals, daß der Antrieb des Querbalkens für die Drehung über den Getriebemotor 44 mit der Rutschkupplung nach Fig. 7 erfolgt. Die Kupplung kann von außen eingestellt werden.

Patentansprüche
----------------------------------

1. Stativ für ein Röntgengerät mit einer vertikalen Säule, an der in vertikaler Richtung verschiebbar ein Vertikalwagen angebracht ist, an dem ein Querbalken drehbar gelagert ist, an dem schwenkbar und in dessen Längsrichtung verschiebbar eine Buckywand und davon getrennt ebenfalls schwenkbar und in dessen Längsrichtung verschiebbar eine Röntgenröhre vorgesehen sind, wobei gegebenenfalls eine wahlweise einschaltbare Steuerung für Antriebe dieser Bauelemente vorgesehen ist, nach Patentanmeldung P 30 10 592.1, d a d u r c h g e k e n n z e i c h n e t , daß die Steuerung die Buckywand (22) in einer einmal eingestellten Höhe hält und gegebenenfalls die Röntgenröhre (26) in eine vorgegebene Position fährt.

2. Stativ nach Anspruch 1, d a d u r c h g e k e n n z e i c h n e t , daß eine Liege (42) für einen zu untersuchenden Patienten an einer seiner Längsseiten offen ist.

3. Stativ nach Anspruch 1 oder 2, d a d u r c h g e k e n n z e i c h n e t , daß der Querbalken (25) manuell in eine gewünschte Schräglage geschwenkt werden kann oder über die Steuerung wahlweise in die horizontale oder vertikale Lage.

4. Stativ nach Anspruch 3, d a d u r c h g e k e n n z e i c h n e t , daß die Steuerung den Vertikalwagen (24) in eine Mittelstellung an der Säule (21) fährt und die Aufnahmedistanz auf einen vorgegebenen Mittelwert.

5. Stativ nach einem der Ansprüche 1 bis 4, d a d u r c h g e k e n n z e i c h n e t , daß die Buckywand (22) über die Steuerung geneigt werden kann.

6. Stativ nach einem der Ansprüche 1 bis 5, d a d u r c h g e k e n n z e i c h n e t , daß im Querwagen (23) der Buckywand (22) ein Lautsprecher (43) angeordnet ist.

7. Stativ nach einem der Ansprüche 1 bis 6, d a d u r c h g e k e n n z e i c h n e t , daß die Röntgenröhre (26) an einem Querwagen (27) befestigt ist, dessen Position über die Steuerung relativ zur Buckywand (22) seitlich einstellbar ist.

8. Stativ nach einem der Ansprüche 1 bis 7, d a d u r c h g e k e n n z e i c h n e t , daß die Höhenlage des Querbalkens (25) über die Steuerung einstellbar ist.

9. Stativ nach den Ansprüchen 1 bis 8, d a d u r c h g e k e n n z e i c h n e t , daß der Drehantrieb (44) für den Querbalken (25) über eine in einem Ölbad laufende Sicherheits-Rutschkupplung erfolgt.

10. Stativ nach einem der Ansprüche 1 bis 9, d a d u r c h g e k e n n z e i c h n e t , daß die Antriebe für die Verschiebungen über einen Getriebemotor (44) mit Kette (45) erfolgt.

FIG 1

0065594

219

FIG 2

FIG 3

FIG 4

FIG 5

FIG 6

FIG 7

46

52

51

50

49

48

47

0065594

8/9

FIG 9

FIG8

FIG 10

**FIG 11**

**FIG 12**

56

56

56

56

44

00065594